# EUROPEAN PATENT SPECIFICATION

(11) **EP 1 082 028 B1**
(45) Date of publication and mention of the grant of the patent: **15.05.2002**
(21) Application number: 98923044.6
(22) Date of filing: 03.06.1998
(51) Int. Cl.: A23L 1/30, A23K 1/16, C10M 159/08, A61K 35/78

(54) **MATTHIOLA SEED OIL, MIXTURES CONTAINING IT AND USE THEREOF**
MATTHIOLASAMEN ÖL, DIESES ENTHALTENDE ZUSAMMENSETZUNGEN UND DEREN VERWENDUNG
HUILE DE GRAINES DE MATTHIOLA, MELANGES LA CONTENANT ET LEUR UTILISATION

(43) Date of publication of application: 14.03.2001
(73) Proprietor: THE STATE OF ISRAEL-MINISTRY OF AGRICULTURE, Beit-Dagan (IL)
(72) Inventor: YANIV, Zohara, 76474 Rehovot (IL); TZUR, Menachem, 75272 Rishon Lezion (IL)
(74) Representative: Söllner, Udo
(86) International application number: IL9800257
(87) International publication number: WO9962359

(56) References cited:
- WO-A-88/10112
- WO-A-93/06812
- US-A- 5 540 762
- YANIV ET AL.: "Evaluation of Matthiola incana as source of omega-3-linolenic acid" INDUSTRIAL CROPS AND PRODUCTS, vol. 6, no. 3-4, 1997, pages 285-289, XP002092061
- YANIV ET AL.: "Differnces in fatty acid composition of oils of wild cruciferae seed" PHYTOCHEMISTRY, vol. 30, no. 3, 1991, pages 841-843, XP002092062

## Description

### Field of the Invention

The present invention relates to Matthiola incana seed oil which is rich in omega 3 linolenic acid, useful as an active ingredient in nutritional supplements for humans and animals. The present invention further relates to use of this oil as a dietary supplement.

### Background of the Invention

Current research in nutritional medicine indicates that the omega - 3- fatty acids are essential components of the human diet. According to studies published in the British scientific journal Lancet, the observed low incidence of arteriosclerosis (fatty plaques development on the inner walls of the arteries which obstructs the blood flow), including coronary artery disease, and chronic inflammatory disease, and diabetes in Greenland Eskimos has been attributed to their traditional ethnic diet, consisting largely of meat from whale, seals, sea birds and fish. This food is rich in fat and protein and low in carbohydrates, but it is extremely high in omega - 3 polyunsaturated fatty acids, especially two omega - 3 fatty acids: C22:6 and C20:5.
The most important Omega - 3 fatty acids are eicosapentaenoic acid (EPA), docosahexaenoic acid (DHA) and α - linolenic acid.
EPA is a direct source of an important substance called prostaglandin E3, which is directly responsible for making blood platelets (cells which form a "plug" in case of injury) less sticky, thus leading to an easier flow of blood. EPA is, therefore, involved in processes that inhibit blood clots, which may obstruct circulation, particularly in the small capillaries of the heart. Of almost equal importance is DHA which comprises a significant amount of the tissues that make up our brains as well as a large part of the retina of the eye.
Some of the most dramatic effects of omega - 3 fatty acids are lowered high blood pressure, reduced serum triglyceride levels and increased clotting time, all positive steps in the prevention of heart and blood vessel diseases. These beneficial effects of omega - 3 fatty acids have been noted in both clinical trials and epidemiological studies.
Omega - 3 fatty acids have also been shown to slow down or prevent cancerous tumor growth, prevent blood vessels from closing following vascular surgery, improve inflammatory diseases such as rheumatoid arthritis and relieve symptoms of psoriasis. Also, omega - 3 fatty acids are essential for proper vision and brain development in newborns.
Omega - 3 fatty acids were found to be extremely useful natural substances powerful enough to normalize the high cholesterol and triglyceride levels that are so extensive in modern population.

The average western diet is low in fresh fish and sea food containing EPA and DHA. On the other hand, it is high in refined carbohydrates and saturated fats. This kind of diet can lead to a serious deficiency in the raw materials necessary for proper platelet function in the blood stream.

Linolenic acid is essential for ensuring healthy skin condition. Moreover, oils containing large amounts of linolenic groups (such as marine oils) were shown to be effective in preventing skin wrinkles and the ingestion of these oils markedly lowers the cholesterol content in the blood. Linolenic acid is a fatty acid found in some plants and can be converted by the body to EPA and DHA. Plant sources of linolenic acid include walnuts and walnut oil, flaxseed, rapeseed (used to make canola oil), soybeans, spinach, mustard greens and purslane.
α - linolenic acid is produced in high quantities by several plants, mainly hemp ( up to 23% α - linolenic acid is pressed from hempseed) and flax (50%). These oils have a bad taste and are not used as edible oils due to their bad taste and smell. Conventional food oils, such as canola and soybean contain only small amounts of linolenic acid (11% and 7% respectively).
Researchers believe that a 1:1 ratio of omega -3 to omega -6 (omega -6 is found primarily in vegetable oils like corn, safflower or sunflower) may be important in preventing heart disease. It seems that omega -3s and omega -6s continually compete for control of important biochemical reactions in the body. When the portion of omega -6 is higher than that of omega -3 it can lead to an overproduction of hormone - like substances called prostaglandins and leukotrienes. Large amounts of these hormone - like substances can disrupt the immune system, initiate the build - up of plaque formations on artery walls, form blood clots and triggers dangerously irregular heart rhythms.
Currently, the ratio in the American diet is about 10 omega -6 to one omega -3 , a ratio, some experts say, which is a dangerous oversupply of omega -6 fatty acids.

Dietary fish oils containing omega -3 fatty acids are increasingly recommended for their antithrombic and hypolipidemic (lowering blood lipid) effects (Phillipson, Rothrock, Connor, Harris and Illingworthm, New England J. Med. 312:1210-16, 1985). Additional benefits of these oils are improvement of immunological function and fighting allergies (Leaf and Weber, New England J. Med. 318:549 - 557, 1988).
Omega - 3 fatty acids from vegetable oils could provide all the above health benefits without any of the disadvantages of oil from animal source.
In ingestion of vegetable oil there is no uptake of cholesterol. Fish oils are a primary source of vitamins A and D. Most marine oils, may supply a potentially toxic amount of vitamins A and D, by supplying a sufficient amount of EPA and DHA factors. Also, vegetal omega - 3 has a good taste as opposed to bad tasting fish oils. Fish oils are usually contained in a natural preservative free gelatin capsule for convenience use due to their bad taste and smell.

International application WO 88/10112 provides a method of increasing the concentration of omega-3 polyunsaturated fatty acids in poultry which comprises administering to the poultry an effective amount of preformed omega-3, polyunsaturated fatty acid or a metabolic precursor thereof. The invention also involves a poultry feed composition useful in effecting this result. Also disclosed is a method of increasing the concentration of omega-3, polyunsaturated fatty acids in poultry eggs which comprises administering to the poultry egg layers an effective amount of preformed omega-3, polyunsaturated fatty acids at a concentration greater than that which naturally occurs or is normally present.

International application WO 93/06812 discloses an emulsion containing one or several polyunsaturated, long chain omega-3 and/or omega-6 fatty acids or their pharmaceutically tolerable esters or salts, as well as usual adjuvants and additives, is used to produce an intravenously administered medicament for treating skin diseases, in particular inflammatory skin diseases as well as diseases of dermatitis or eczema group.

U.S. patent No. 5540762 discloses a method of preventing spontaneous combustion of a transparent composition containing unsaturated drying oil including a glyceride of an unsaturated acid. Formulations containing certain unsaturated drying oils, e.g., linseed oil, are rendered flame retardant by dissolving in them certain halogenated compounds which are solids at room temperature and soluble in linseed oil or other suitable solvents which can be added to the linseed oil formulations with phosphate-containing compounds to enhance flame retardance.

Z. Yaniv *et al.* (Z. Yaniv, D. Schafferman, M. Zur and I. Shamir) described a hot extraction method for extraction of Mattiola oil. Seed oil of selected lines of Matthiola incana (Cruciferae) have an average oil content of 20 - 24 % and are a rich source of omega - 3- linolenic acid (55 - 65%) (Yaniv, Shamir, Shefferman and Zur, Proc. 3rd. Nat. Sym. New Crops, Indiana USA. This is the highest known content of linolenic acid in plant species. The other components of this oil are two important fatty acids: Oleic acid (C18:1) and linoleic acid (C18:2). Both acids are unsaturated fatty acids and are essential in the human diet.
Another important aspect of this vegetable oil is its quality as drying oil, for painting and lubrication, due to the high content of polyunsaturated fatty acids , namely linolenic acid. Up to date, vegetative drying oil is obtained from crops such as flax seeds and Tong trees. These crops do not lend themselves to mechanical harvesting and cleaning.

The present invention relates to an oil obtained from Matthiola incana seeds, having all the health benefits of fish oil but none of its drawbacks, and having the additional above mentioned benefits over other vegetable oils, such as for dietary supplements.

### Summary of the Invention

The present invention relates to Matthiola incana seeds oil as an active ingredient in dietary supplements.

The present invention further relates to a method for obtaining oil from Matthiola incana seeds comprising pressing of the plant seeds, collecting the resulting oil and purifying it and also to a method for the preparation of compositions containing Matthiola incana seeds oil comprising pressing Matthiola incana seeds, collecting the resulting oil, purifying it and mixing resulting oil with acceptable additives.
Further, the present invention relates to the use of an oil, as prepared according to the above method, for the production of a dictary supplement.

### Brief description to drawing

Figure 1 describes the mean temperature (°C) measured at Bet Dagan, Jerusalem and Ramat Hagolan Experiment station, during the 1991-1992 growing season.

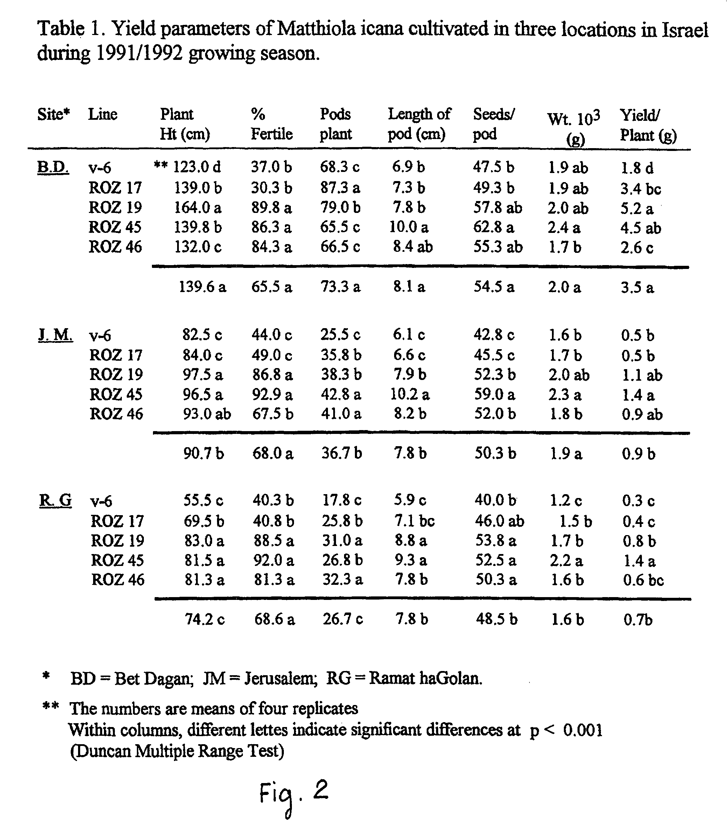

Figure 2 describes the yield parameter of Matthiola icana cultivated in three locations in Israel during 1991/1992 growing season.

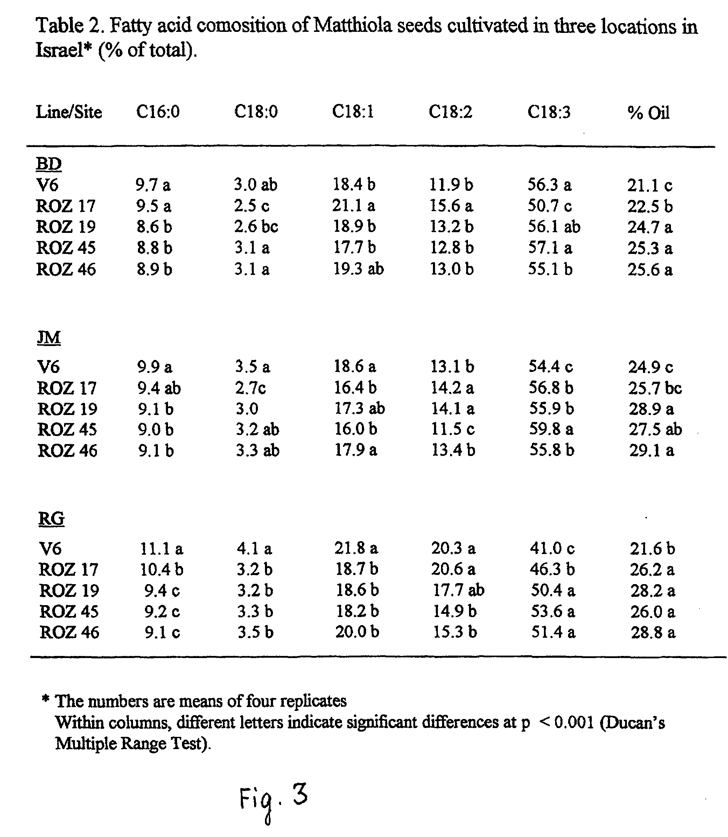

Figure 3 describes fatty acid composition of Matthiola seeds cultivated in three locations on Israel.

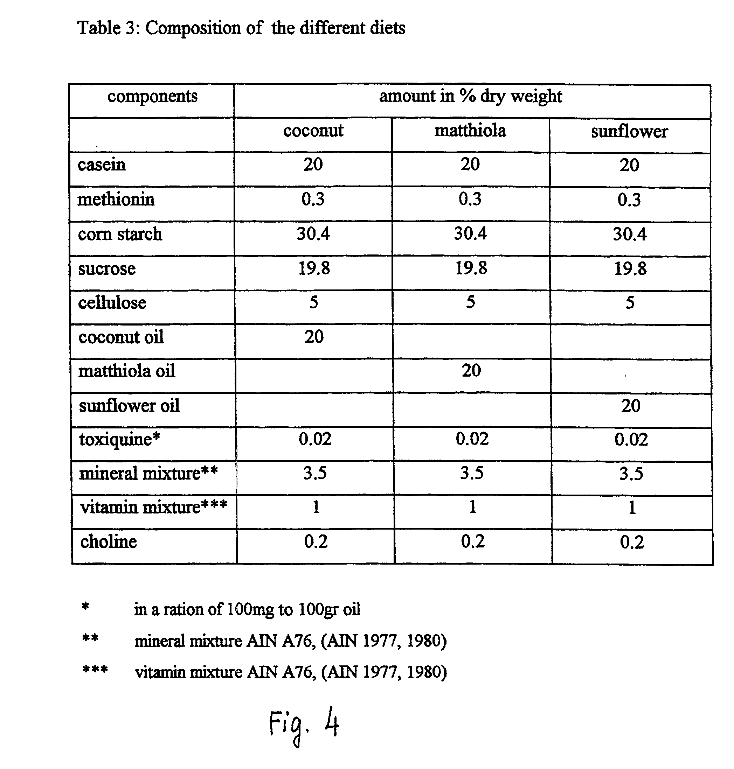

Figure 4 describes composition of the different diets.

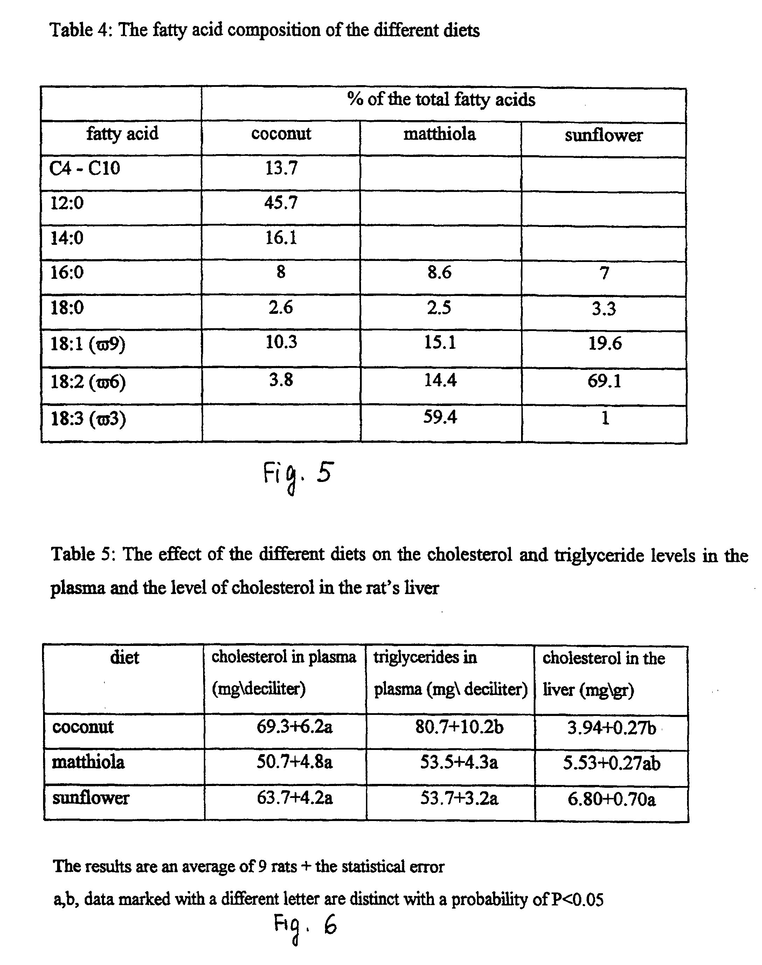

Figure 5 describes the fatty acid composition of the different diets.
Figure 6 describes the effect of the different diets on the cholesterol and triglyceride levels in the plasma and the level of cholesterol in the rat's liver.

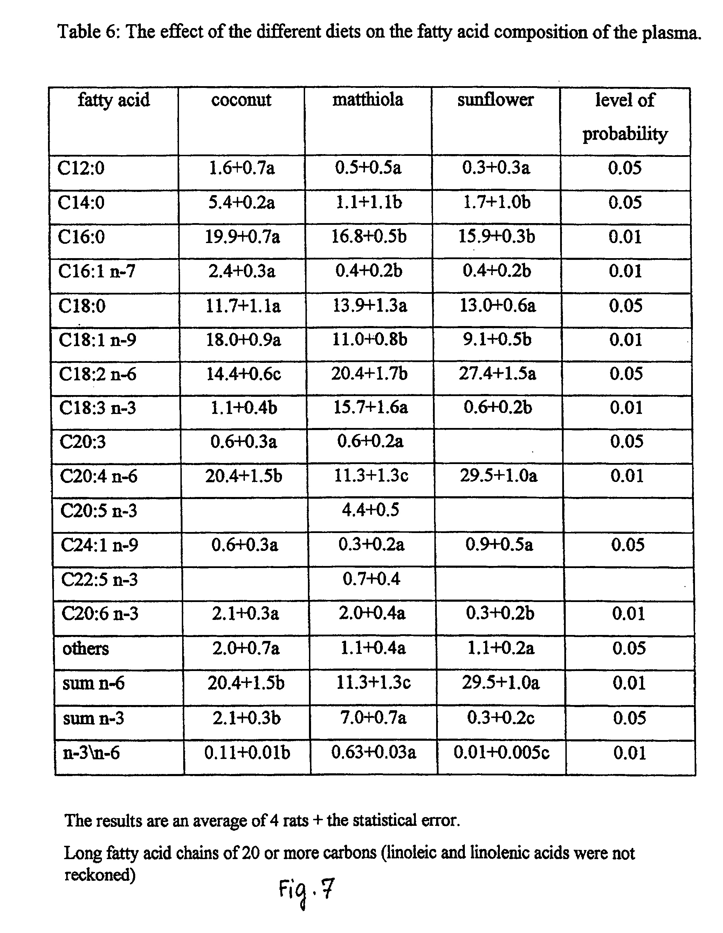

Figure 7 describes the effect of the different diets on the fatty acid composition of the plasma.

### Detailed Description of the Invention

Matthiola incana has an average oil content of 20 - 24% in the seeds, with maximum levels of 68% omega - 3 - linolenic acid of the total fatty acids in the oil (Ecker, Yaniv, Zur and Shaffermann, Euphytica 59:93-93, 1992). This is the highest known content of linolenic acid in plant species.
Matthiola lines were tested and evaluated as a potential new oil crop for dietary supplement for humans and animals, for use as an active ingredient in pharmaceutical and cosmetic compositions and mixtures and for industrial uses.
Omega - 3 fatty acids from vegetable oils could provide all the above health and cosmetic benefits without any of the disadvantages of oil from animal source.
In ingestion of vegetable oil there is no uptake of cholesterol. Fish oils are a primary source of vitamins A and D. Most marine oils, may supply a potentially toxic amount of vitamins A and D, by supplying a sufficient amount of EPA and DHA factors. Also, vegetal omega - 3 has a good taste and odor as opposed to bad tasting and foul smelling fish oils. Fish oils are usually contained in a natural preservative free gelatin capsule for convenience use due to their bad taste and smell.
Another important aspect of this vegetable oil is its quality as drying oil, for painting and lubrication, due to the high content of polyunsaturated fatty acids , namely linolenic acid. Up to date, vegetative drying oil is obtained from crops such as flax seeds and Tong trees. These crops do not lend themselves to mechanical harvesting and cleaning.

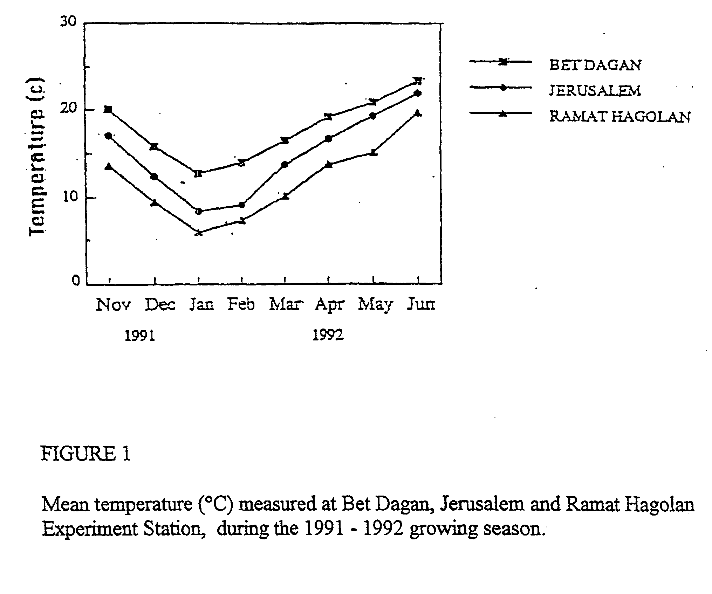

The present invention will be further illustrated in detail by the following experiments.
These experiments do not intend to limit the scope of the invention but to demonstrate and clarify it only.
1) Five lines of M. incana, with a high content of omega - 3 - linolenic acid, were tested. Plants were grown in the experimental stations at Bet Dagan, Ramat haGolan and Jerusalem, representing three different climatic regions, during the 1991\92 growing season. Each line was replicated four times in random block design. Seeds of each line were sown in small plots of 1.2 m² consisting of four rows with 30 cm between rows. Basic fertilization was conducted at the time of soil preparation, at rates of 100\100\50 N\P\K. "Trifluralin (2500 g\ha) was used as a herbicide. Irrigation was applied until seedling establishment. data concerning mean monthly temperatures of the growing period (Nov. 91 - June 92) taken in the three sites is presented in figure 1.
   Observations were made on plant height, percent of fertile plants and seed yield parameters such as number of pods\plant, length of pod, number of seed\pod and weight of 10³ seeds. Plants were harvested at each location according to their dates of full maturity. Fully mature seeds from each line were oven - dried overnight at 50°c and analyzed for oil content and fatty acid composition.
   Lipid extraction - Seeds were dried overnight at 50°c and ground into powder in a Moulinex® coffee grinder. Five grams of powder were mixed with 100 cc petroleum ether (40 - 60°c), and the lipid fraction was extracted in a Soxhlet apparatus for 16 hours at 60°c. The solvent was evaporated and the lipid fraction residues were weighed.
   Direct transesterification from seeds - Seeds (200 mg) were dried overnight at 50°c and ground into powder with a mortar and pestle, after which 0.3 cm³ of dichloromethane and 2 cm³ of 0.5N sodium methoxide (MeONa) were added. The tube was shaken and heated for 30 minutes at 50°c. The reaction was stopped by adding 5 cm³ of water containing 1 cm³ glacial acetic acid. The esterified fatty acids were extracted with 2 cm³ petroleum ether ( 40- 60°c). The clear fraction was kept at -20°c until further analysis. Samples of 2 mm³ were injected into the gas - chromatograph for fatty acid analysis.
   Gas chromatography of methylated fatty acids - A Megabore column (DB-23, 0.5 µm film thickness, 30m x 0.54mm, J&W Scientific) was used in a gas chromatograph equipped with a flame ionization detector (Varian, model 3700GC) and an automatic area integrator (3390A HP). The flow rate of N2 (carrier gas) was 30 cc/min and the oven temperature was 135 - 200°c, programmed at a rate of 4°c min. The following fatty acids were identified using known standards (Supelco): C16:0, palmitic; C18:0, stearic; C18:1, oleic; C18:2, linoleic C18:3, linolenic; C20:1, eicosenoic; and C22:1 erucic acid.
   Table 1 summarizes the results of the evaluation experiment of five lines of M. incana cultivated in three places in Israel Bet Dagan (BD), Jersusalem (JM) and Ramat haGolan (RG) experimental stations during fall 1991 - spring 1992. Yield parameters, such as yield per plant, wt. of 10³ seeds, no. of seeds\pod, pod length, plant height and no. of pods per plant were measured.
   The results indicate the following:
   (a) the best location was Bet Dagan. All lines tested showed higher yield potential. Yield per plant was by far the highest (3.5 g/plant as compared with 0.9 g/plant in JM 0.7 g/plant in RG). Other outstanding results are the number of pods/plants (73.3 in BD as compared with 36.7 in JM and 26.7 in RG). The number of seeds/pod, plant ht., pod length and the wt. of 10³ seeds were also slightly higher in BD as compared with the other sites.
   (b) the best performing lines were ROZ 45 and ROZ 19. At BD both lines showed the highest yield potential. Under low temperature conditions in JM and RG both lines ROZ 45 in particular, maintained the highest yield, even though it was lower than in BD. It should be stressed that both lines have the highest (close to 90%) fertility rate in all sites tested.
   Oil chemistry - Table 2 summarizes the results of the evaluation data concerning oil content and fatty acid composition of seeds of Matthiola lines harvested at the three sites. The figures represent means of four replicates.
   Oil quantity - oil quantity ranged from 21% (V6) to 28- 29% (ROZ 19 and ROZ 46), the best sites for this parameter being Jerusalem and Ramat haGolan. Seeds of both ROZ 46 and ROZ 19 accumulated 29% oil in JM and RG as compared with 25% oil at BD. In these two sites the temperatures during seed maturation were lower than in BD (fig. 1). It is known that low temperatures during seed development have a positive effect on oil quantity. It could be that this difference in oil quantity of Matthiola seeds obtained in the three sites is due to the difference in temperatures.
   Fatty acid composition - The main goal is obtaining a high concentration of α - linolenic acid (C18:3) in the seed oil. The obtained levels ranged from 50% to 60%, the best line being ROZ 45 at all three locations. The best locations were JM and BD. Temperature plays a major role in the relative concentration of unsaturated fatty acids of seed oils. Usually, cooler conditions favor the production of polyunsaturated fatty acids. When Matthiola seeds were cultivated under controlled temperature conditions in the Phytotron, the content of unsaturated linolenic acid of seeds grown at lower temperatures (12°C\17°C) was much higher (69%) than that of seeds maturing at warmer temperatures (58% of C18:3 at 22°C\27°C). However, in spite of the fact that the coolest temperatures prevailing during seed maturation were at the RG site (fig. 1), the highest content of C18:3 was obtained at JM and BD site, and it did not reach beyond 60% (table 2).
   It is important to note that these temperatures were never below 22°C during the maturation period (March - May). It could be that in order to induce a significant increase in the level of C18:3, maturation should take place under a much cooler temperature regime, as demonstrated in Phytotron conditions (Yaniv et al. Israel J. Bot. 41:279 - 284, 1992).
   In conclusion, the biometric recordings revealed that the BD site induces the best preformance. Since oil content and α - linolenic acid contents are not much lower than at other sites, the final yield, in terms of α - linolenic acid \ plant, is significantly the highest at the site of BD.
   Both ROZ 19 and ROZ 45 excel in yield parameters, have close to 90% fertility and have the highest oil quantity and highest content of α - linolenic acid.
   A calculated yield of 750kg seeds/ha was obtained at BD. This is based on density of 17 plants \m². This yield, with 20 - 25% oil in the seed is equal to 150 l oil/ha. A 50% content of α - linolenic acid in the oil will yield 75 liters of pure α - linolenic acid from 1 hectar of the crop.
2) Hens were given a diet consisting of 1% Matthiola incana seed oil and the amount of two omega - 3 fatty acids (C22:6 and C18:3) in the eggs laid by these hens was established.
   The results show that C22:6 (eicosapentaenoic acid) contents in the eggs laid by the hens with the Matthiola oil diet was 2.4% of the fatty acids of the egg, as opposed to 1.5% in eggs laid by control hens.
   C18:3 contents in the hens with the Matthiola oil diet was 2.2% as opposed to 0% in the control hen's eggs.
   All together, the omega - 3 fatty acid content of the treated hen's eggs were 4.9% as opposed to 1.4% in control hen's eggs.
3) 27 male rats (of the type Sprague Dawly) were devided to three experimental groups according to the different diet each group received. The rat's weight was in the range of 175 - 205gr and they were kept in a controlled environment (light from 6 a.m to 6 p.m and a temperature of 22 - 24°C) for 6 weeks. The rats had free access to food and water. Fresh food was supplied every two days. The rats were weighed once a week and their food consumption was assested every two days.
   One group received a diet containing coconut oil, the second group received a Matthiola containg diet and the third group received a sunflower oil containing diet. A mixture of all the food ingredients except the oil was prepared once a week and stored at -20°C. The oil was added to the mixtures every two days to achieve maximal freshness and to avoid oxidation.
   The compositions of the different diets given in the experiment are shown in table 3 and table 4 summarizes the fatty acid composition of the oils given in the different diets.
   Blood samples were taken from the rat's tails after a night's fast and the samples were left in heparin washed tubes overnight to dry. The samples were centrifuged and the plasma collected and kept at -20°C.
   Animals were killed in three succesive days so that 2-4 animals of each group were killed a day. The animal's spleen and liver were removed and weighed. The liver was immediately dry freezed and kept at -70°C.
   Determining the triglyceride level in the plasma - The triglyceride levels in the animal's plasma were determined enzymatically using the Raichem® kit (San Diego, California) which contains lipases that perform hydrolysis of triglycerides to glycerol and free fatty acids. The glycerol is phosphorylated and oxidized and the resulting peroxide is reacted with 4- aminoantipyrin and 3,5 - dichloro-2-hydroxybenzene sulfonate to give a red color. This color's absorbency in 520 nm is proportional to the triglyceride concentration in the sample.
   Determining the cholesterol level in the plasma - Cholesterol levels in the plasma were determined enzymatically using the Raichem® kit (San Diego, California) which contains cholesterol esters that are hydrolyzed by cholesterol esterases to give cholesterol and free fatty acids. The free cholesterol is oxidized and the resulting hydroproxide, together with a peroxidase, oxidizes P- hydroxybenzoate and 4 - aminoantipyrin to give a red color. This color's absorbency at 500 nm is proportional to the cholesterol concentration in the sample.
   Determining the cholesterol level in the liver - The cholesterol levels in the liver were determined using the method described in Searcy & Bergquest (Clin. Chem. Acta 5: 192 - 199, 1960) with the following differences: Hepatadecaenoic acid (C 17:0) in ethanol was added to each sample as an intrinsic standard for determining the fatty acid content of the liver. KOH 10% was added to the samples and the samples were incubated at 65°C for an hour. The samples were extracted with petrol ether.
   For determining the cholesterol levels the samples were dried and saturated acetic acid in FeSO4 was added to acheive a color reaction. The results were read at 490 nm.
   Determining the fatty acid composition in the liver - Methyl red was added to the fractions left after the petrol ether extraction and the mixture was titrated to give a stable pink color. The fatty acids were extracted with petrol ether and were methylated. The fatty acid composition of the resulting methyl esters was determined using the method described for direct transesterification from seed (Experiment 1).
   Determining the fatty acid composition in the plasma - Hepatadecaenoic acid (C 17:0) in chloroform was added to each sample as an intrinsic standard for determining the fatty acid content of the liver. The sample was extracted with chloroform\ ethanol. The extracted fatty acids were methylated. The fatty acid composition of the resulting methylated fatty acids was determined using the method described for direct transesterification from seed (Experiment 1).
   Determination of oxidation in the liver: Oxidation in the liver was determined using thiobarbitituric acid which forms a color reaction with melon aldehyd. Melon aldehyd is a compound which is the oxidation product of a system containing a large amount of unsaturated fatty acids undergoing oxidation. The method using TBA utilizes the color reaction that occurs in the presence of TBA.
   The results were read at 535 nm.

Table 5 shows the effect the different diets have on the cholesterol and triglyceride levels in the plasma and on the cholesterol level in the liver.
From table 5 it is apparent that the cholesterol levels in the plasma of Matthiola oil fed animals was lower than that of the animals fed with coconut oil and also than those animals fed with sunflower oil.
The triglyceride levels of animals fed with Matthiola oil and sunflower oil are lower than those animals fed with coconut oil.
The cholesterol levels in the liver of the coconut oil fed animals was significantly lower than that in the liver of animals fed with sunflower oil. The cholesterol level in the liver of animals fed with Matthiola oil was not significantly different than the other animals.

Table 6 shows the fatty acid content in the rat's plasma. The coconut oil diet raised the levels of C16:1, C16:0 and C14:0 in the plasma compared with the other two diets. There was no difference in the C18:0 and C12:0 levels between the different diets.
The oleic acid (C18:1) was highest in the plasma of rats fed with sunflower oil, lower in the Matthiola oil fed rats and lowest in the coconut oil fed rats.
The α - linolenic acid level was significantly higher in the plasma of rats fed with Matthiola oil (15.7%) than the other two diets (0.6% sunflower oil and 1.1% coconut oil, P< 0.01). The archidonic acid levels (C20:4 n-6) were highest in the sunflower oil diet (29.5%), lower in the coconut oil diet (20.4%) and lowest in the mathiola oil diet (11.3%) (P<0.01).
The long fatty acid levels from the group of n-3 was significantly higher in the plasma of those animals fed with Matthiola oil compared with the other two diets. EPA (C20:5 n-3 and C22:5 n-3) appeared only in the plasma of the animals fed with Matthiola oil (4.4% and 0.7% respectively). DHA (C22:6 n-3) appeared in the plasma of animals fed with Matthiola and cocnut oil more than in the sunflower diet animals (12% and 2.1% compared with 0.3% P<0.01).
Table 6 shows that the sum of the long fatty acids (over 20 carbons) from the n-6 group is very high in the plasma of animals fed with sunflower oil (29.5%), lower in the coconut oil diet (20.4%) and very low in the Matthiola oil diet (11.3%) (P<0.01). In contrast, the sum of the long fatty acids from the n-3 group was high in the Matthiola diet (7.0%), lower in the coconut diet (2.1%) and lowest in the sunflower oil diet (0.3%).

These experiments demonstrate that the α - linolenic acid content of the oil in Matthiola incana seeds is high, the plants are easily grown and the oil is easily obtained. Moreover, when fed to animals that provide food products for human consumption, such as eggs from hen, Matthiola incana seed oil raises the omega 3 fatty acid contents of these food products.
Animals fed with a diet enriched in Matthiola seed oil have lower cholesterol levels in their plasma and higher amounts of α - linolenic acid, EPA and DHA.
The present invention, therefore, relates to an easily obtained vegetable oil useful as an active ingredient in nutritional supplements for humans and animals, pharmaceutical and cosmetic compositions and mixtures and mixtures for industrial use.

## Claims

1. A method for obtaining oil from Matthiola incana seeds comprising pressing of the plant seeds, collecting the resulting oil and purifying it.

2. A method for the preparation of compositions containing Matthiola incana seeds oil comprising pressing Matthiola incana seeds, collecting the resulting oil, purifying it and mixing resulting oil with acceptable additives.

3. Use of an oil as prepared according to the method of claims 1 or 2 for the production of a dietary supplement.

## Patentansprüche

1. Verfahren zur Gewinnung von Öl aus Samen von Matthiola Incana, beinhaltend Pressen der Pflanzensamen, Auffangen des resultierenden Öls und dessen Aufreinigung.

2. Verfahren zur Herstellung von Zusammensetzungen, welche Samenöl von Matthiola Incana enthalten, beinhaltend Pressen von Matthiol Incana Samen, Auffangen des resultierenden Öls, dessen Aufreinigung und Vermengen resultierenden Öls mit tauglichen Zusatzsstoffen.

3. Verwendung eines Öls, hergestellt nach einem Verfahren gemäss Anspruch 1 oder 2 für die Herstellung eines Diätzusatzes.

## Revendications

1. Méthode pour obtenir de l'huile de graines de Matthiola Incana comprenant pressant les graines, récupérant l'huile résultante et la purifier.

2. Méthode pour la préparation de compositions contenant de l'huile de graines de Matthiola Incana, comprenant pressant les graines de Matthiola Incana, récupérant l'huile résultante, la purifiant et mélangeant l'huile résultante avec des additifs acceptables.

3. Usage d'une huile préparée selon un méthode des revendications 1 ou 2 pour la production d'un supplément diététique.
